Europäisches Patentamt

(19) European Patent Office

Office européen des brevets

(11) EP 0 971 739 B1

(12) **EUROPEAN PATENT SPECIFICATION**

(45) Date of publication and mention
of the grant of the patent:
**06.10.2004 Bulletin 2004/41**

(21) Application number: **98915239.2**

(22) Date of filing: **01.04.1998**

(51) Int Cl.⁷: $A61K\ 39/39$

(86) International application number:
**PCT/US1998/006528**

(87) International publication number:
**WO 1998/043670 (08.10.1998 Gazette 1998/40)**

(54) **AQUEOUS IMMUNOLOGIC ADJUVANT COMPOSITIONS OF MONOPHOSPHORYL LIPID A**

WÄSSERIGE IMMUNOLOGISCHE ADJUVANTZUSAMMENSETZUNGEN AUS MONOPHOSPHORYLLIPID A

COMPOSITIONS D'ADJUVANT IMMUNOLOGIQUE AQUEUSES DE MONOPHOSPHORYLE LIPIDE A

(84) Designated Contracting States:
**AT BE CH CY DE DK ES FI FR GB GR IE IT LI LU MC NL PT SE**

(30) Priority: **01.04.1997 US 831073**

(43) Date of publication of application:
**19.01.2000 Bulletin 2000/03**

(73) Proprietor: **CORIXA CORPORATION**
**Seattle, WA 98104 (US)**

(72) Inventor: **CRANE, R., Thomas**
**Hamilton, MT 59840 (US)**

(74) Representative: **Couchman, Jonathan Hugh et al**
**Harrison Goddard Foote,**
**Belgrave Hall**
**Belgrave Street**
**Leeds LS2 8DD (GB)**

(56) References cited:
**WO-A-94/21292**      **GB-A- 2 220 211**

- C. BOECKLER ET AL.: "Immunogenicity of new heterobifunctional cross-linking reagents used in the conjugation of synthetic peptides to liposomes." JOURNAL OF IMMUNOLOGICAL METHODS, vol. 191, no. 1, 10 May 1996, pages 1-10, XP002075734 Amsterdam, The Netherlands
- A. VERHEUL ET AL.: "Beneficial effects of additional adjuvants on the immune response to haptenated liposomes." JOURNAL OF LIPOSOME RESEARCH, vol. 6, no. 2, 1996, pages 397-414, XP002075735 New York, NY, USA
- S. SASAKI ET AL.: "Monophosphoryl lipid A enhances both humoral and cell-mediated immune responses to DNA vaccination against human immunodeficiency virus type 1." INFECTION AND IMMUNITY, vol. 65, no. 9, September 1997, pages 3520-3528, XP002075736 Washington, DC, USA

**Description**

Background of the Invention

The compounds monophosphoryl lipid A (MLA) and 3-O-deacylated monophosphoryl lipid A (3D-MLA) are attenuated derivatives of the lipid A component of bacterial lipopolysaccharide (LPS). LPS and lipid A are potent immunostimulants inducing both a humoral antibody response and a cell-mediated immune response in patients administered the compounds. Lipid A and LPS however can also display toxic side-effects such as pyrogenicity and local Shwarzman reactions. MLA and 3D-MLA are lipid A-like molecules that have been modified to attenuate the toxicity of LPS.

Like lipid A, the MLA and 3D-MLA molecules have a sugar backbone onto which long chain fatty acids are attached. The backbone is comprised of two six carbon sugar rings in glycosidic linkage. MLA and 3D-MLA are phosphorylated at the 4 position. Five to eight long chain fatty acids (12-14 carbons) are attached to the sugar backbone making MLA and 3D-MLA very hydrophobic molecules which are not readily water soluble.

The attenuated lipid A derivatives (ALDs) MLA and 3D-MLA are used as immunologic adjuvants in prophylactic vaccines for infectious disease and therapeutic vaccines for the treatment of cancerous tumors and chronic infections. Antigen preparations included in most vaccines are often complicated mixtures of water-soluble proteins making it difficult to formulate the water insoluble adjuvant in a water based vaccine. Therefore, MLA and 3D-MLA must be first mixed with solvents before they are added to the antigen preparation. However, the presence of solvents can further complicate the formulation of the vaccine, and in some cases can reduce the efficiency of its components. Further, solvents can irritate mucosal surfaces or cause inflammation at an injection site. A simple formulation of MLA or 3D-MLA containing no interfering co-soivents would allow maximum benefits to be derived from both the adjuvant and the antigen in a vaccine composition. The instant invention satisfies this need.

WO-A-94/21292 describes a vaccine composition comprising an antigen in conjunction with 3-O-deacylated monophosphoryl lipid A (which may be abbreviated to "3D-MPL" or 3D-MLA") and a suitable carrier wherein the particle size does not exceed 120nm. The carrier may be an oil-in-water emulsion, a lipid vehicle or aluminium hydroxide; the emulsions may contain an emulsifier.

GB-A-2220211 and equivalent US-A-4912094 disclose the production of 3D-MLA as described in more detail hereinafter with references to the US patent. These documents teach that 3D-MLA and monophosphoryl lipid A (which may be abbreviated to "MPL" or "MLA") may be used as an adjuvant, and that 3D-MLA may be administered as a vaccine in saline, in a lipid emulsion or in an oil-in-water emulsion containing surfactant (Tween®80). The dispersion of MLA and 3D-MLA in sterile water containing triethylamine is described, using warming and an ultrasonic bath to promote solubilisation.

C. Boeckler et al. "Immunogenicity of new heterobifunctional cross-linking reagents used in the conjugation of synthetic peptides to liposomes" (1996) J. Immunol. Methods 191, 1-10, describe an investigation into the immunogenicity of six thiol-reactive cross-linking reagents. There is described the preparation of liposome-linked peptides, in which small unilamellar vesicles were prepared from phosphatidylcholine, phospatidylglycerol, (p-maleimidophenyl) butyrylphosphatidylethanolamine and cholesterol. The lipids in chloroform solution with MLA were dried under high vacuum and resuspended in buffer by vortexing and sonication. The liposome suspension was then further processed.

Summary of the Invention

The subject invention relates to an aqueous formulation of adjuvant consisting of an attenuated lipid A derivative (ALD), and a surfactant, as well as to methods for its preparation. The ALD and the surfactant are in water free of co-solvent. The attenuated lipid A derivatives are monophosphoryl lipid A (MLA) or 3-O-deacylated monophosphoryl lipid A (3D-MLA); the molar ratio of the ALD to the surfactant is from 10:1 to 2:1. Such aqueous formulations of MLA (MLA/AF) or 3D-MLA (3D-MLA/AF) eliminate the need for undesirable solvents or a co-solvent system for vaccine preparation. The invention provides a stable aqueous composition of the ALD and a surfactant which, when administered to mice with an antigen, enhances the cellular and humoral immune response of the animal to that antigen. Surprisingly, the aqueous formulation of the present invention induces high levels of serum and mucosal secreted IgA in immunized animals when administered intranasally. An embodiment of the claimed aqueous composition comprises a MLA or 3D-MLA to surfactant molar ratio of about 4:1 and has a particle size of approximately 50-70 nm. 1,2-Dipalmitoyl-sn-glycero-3-phosphocholine (DPPC) is a preferred surfactant.

A method of preparing the aqueous composition is disclosed. In one embodiment the ALD and the surfactant are dissolved and uniformly admixed in ethanol. The ethanol is then evaporated leaving a film. Water is added to the film. The ALD and surfactant are suspended in the water by sonication. The suspension is sonicated until clear. Animals administered the claimed composition with an antigen display enhanced humoral and cellular immune responses to that antigen. Methods for using the composition to enhance these responses are also disclosed. Other aspects and embodiments of the invention are set forth in the claims.

Brief Description of the Figures

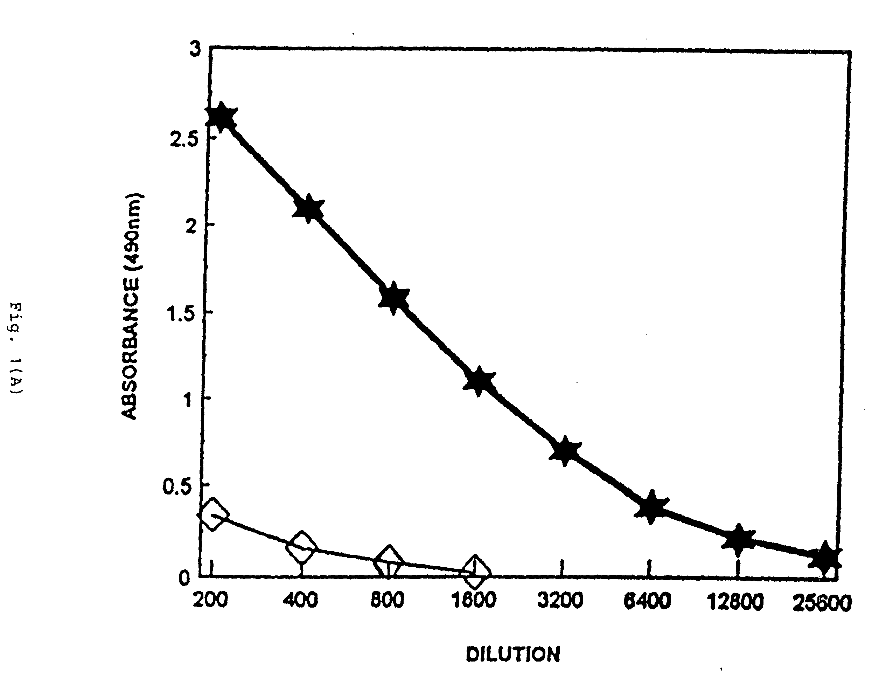

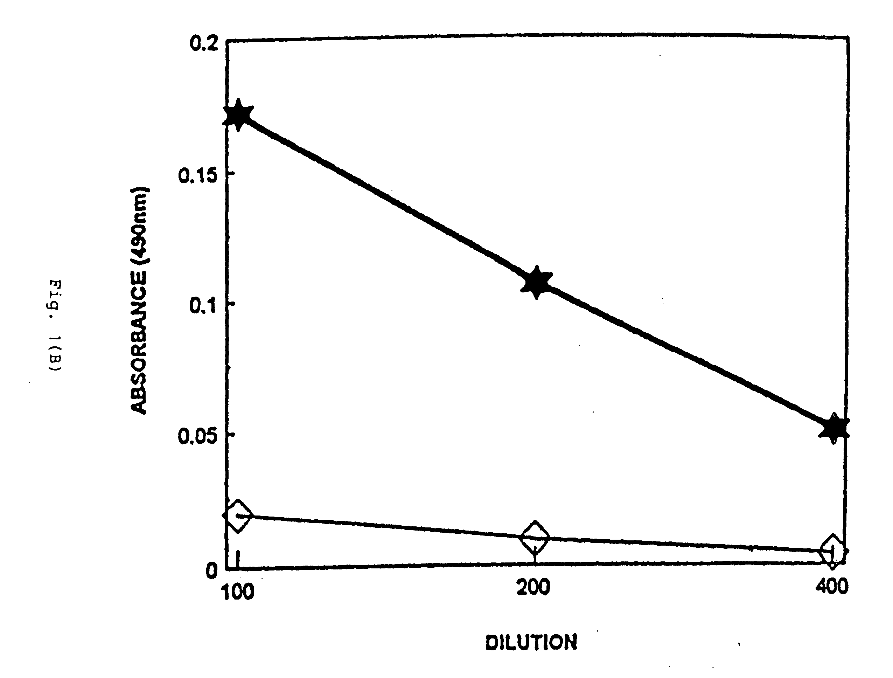

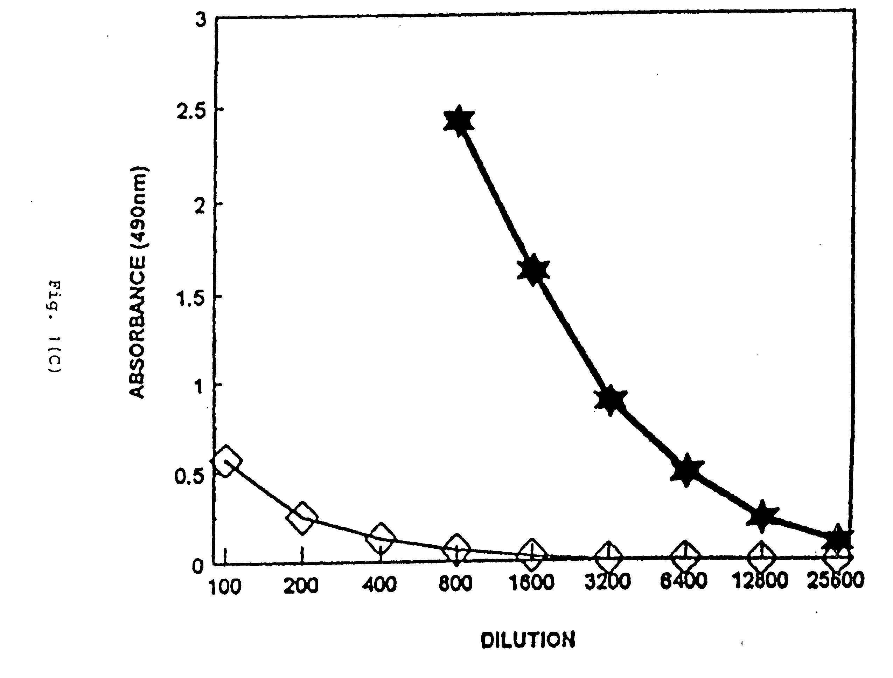

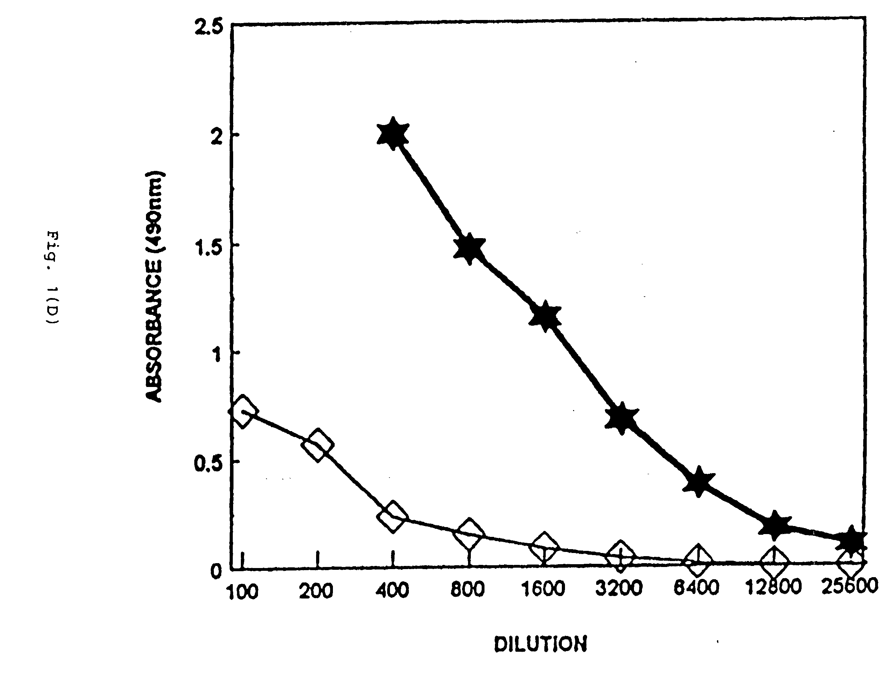

**Figure 1 a-d** show the antibody titers of mice administered tetanus toxoid (TT) antigen in 3-O-deacylated monophosphoryl lipid A-aqueous formula (3D-MLA/AF) * or tetanus toxoid antigen in saline ♦. Figure la shows the total IgG antibody titers of mice administered the tetanus toxoid antigen. Figure 1b shows the IgG2a antibody titers of mice administered the tetanus toxoid antigen. Figure 1c shows the IgG2b antibody titers of mice administered the tetanus toxoid antigen and Figure 1d shows the IgG1 antibody titers for the animals.

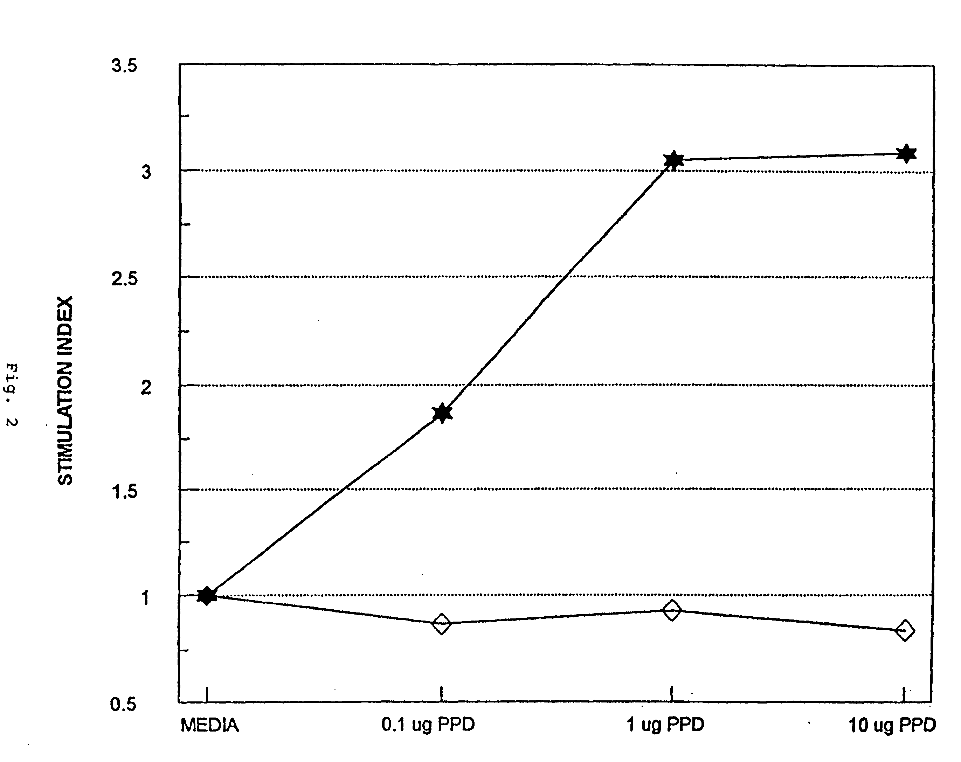

**Figure 2** shows the T-cell proliferative response in mice immunized with a purified protein derivative. The proliferative response in mice administered tetanus toxoid in 3D-MLA/AF ∗ and normal controls ♦ are shown 14 days post primary vaccination.

Detailed Description of the Invention

The subject invention involves an aqueous adjuvant formulation of an attenuated lipid A derivative (ALD). In one embodiment, the ALD and a surfactant are suspended in water in a molar ratio of approximately 4:1 and sonicated to yield a suspension having a particle size of approximately 50-70 nm.
In accordance with the present invention, an attenuated lipid A derivative can be formulated into an aqueous composition to provide a potent adjuvant. An attenuated lipid A derivative is a lipid A-like compound which displays the advantageous immunostimulatory properties of lipid A yet exhibits less of the adverse side affects of that compound. The compositions of the invention comprise an ALD selected from monophosphoryl lipid A (MLA) and 3-o-deacylated monophosphoryl lipid A (3D-MLA), which are ALDs that are potent immunostimulants but are surprisingly less toxic than lipid A. Both MLA and 3D-MLA can be used in the compositions of the subject invention and are known and need not be described in detail herein. See for example U.S. Patent No 4,436,727 issued March 13, 1984, assigned to Ribi ImmunoChem Research, Inc., which discloses monophosphoryl lipid A and its manufacture. U.S. Patent No. 4,912,094 and reexamination certificate B1 4,912,094 to Myers, *et al.,* also assigned to Ribi ImmunoChem Research, Inc., embodies 3-O-deacylated monophosphoryl lipid A and a method for its manufacture.
Without going into the details of the above prior patents, monophosphoryl lipid A (MLA) as used herein is derived from lipid A, a component of enterobacterial lipopolysaccharides (LPS), a paotent but highly toxic immune system modulator. Edgar Ribi and his associates achieved the production of monophosphoryl lipid A (MLA) referred to originally as refined detoxified endotoxin. MLA is produced by refluxing an endotoxin extract (LPS or lipid A) obtained from heptoseless mutants of gram-negative bacteria in mineral acid solutions of moderate strength (e.g. 0.1 N HCl) for a period of approximately 30 minutes. This treatment results in the loss of the phosphate moiety at position 1 of the reducing end glucosamine.
Coincidentally, the core carbohydrate is removed from the 6 position of the non-reducing glucosamine during this treatment. The resulting product (MLA) exhibits considerably attenuated levels of the endotoxic activities normally associated with the endotoxin starting material, such as pyrogenicity, local Shwarzman reactivity, and toxicity as evaluated in the chick embryo 50% lethal dose assay ($CELD_{50}$). However, it unexpectedly retains the functionality of lipid A and LPS as an immunomodulator.
3D-MLA is known as set forth in U.S. Patent No. 4,912,094, reexamination certificate B 14,912,094 (the '094 patent), and differs from MLA in that there is selectively removed from the MLA molecule the β-hydroxymyristic acyl residue that is ester linked to the reducing-end glucosamine at position 3 under conditions that do not adversely affect the other groups. 3-O-deacylated monophosphoryl lipid A is available from Ribi ImmunoChem Research, Inc., Hamilton, Montana 59840.
The MLA and 3D-MLA molecules are a composite or mixture of a number of fatty acid substitution patterns, i.e., heptaacyl, bexaacyl, pentaacyl, etc., with varying fatty acid chain lengths. Thus, these various forms of MLA and 3D-MLA are encompassed by this invention. Further, mixtures of forms of a compound as well as individual compounds produced by synthetic or semisynthetic means are encompassed by this invention. The lipid A backbone that is illustrated in the --094 patent corresponds to the product that is obtained by 3-deacylation of heptaacyl lipid A from *S. minnesota* R 595. Other fatty acid substitution patterns are encompassed by this disclosure; the essential feature is that the material be 3-O-deacylated.
The 3D-MLA utilized in the present invention is prepared by subjecting MLA to alkaline hydrolysis under conditions that result in the loss of but a single fatty acid from position 3 of the lipid A backbone. β-hydroxymyristic fatty acid at position 3. is unusually labile in alkaline media. It requires only very mild alkaline treatment to completely 3-deacylate lipid A. The other ester linkages in lipid A require somewhat stronger conditions before hydrolysis will occur so that it is possible to selectively deacylate these materials at position 3 without significantly affecting the rest of the molecule. The reason for the unusual sensitivity to alkaline media of the ester-linked β-hydroxymyristic fatty acid at position 3 is not known at this time.

Although alkaline hydrolysis procedures are known, it is important to choose conditions that do not cause further hydrolysis beyond the ester linkage to the β-hydroxymyristic at position 3. In general the hydrolysis can be carried out in aqueous or organic media. In the latter case, solvents include methanol (alcohols), dimethyl sulfoxide (DMSO), dimethylformamide (DMF), chloroform, dichloromethane, and the like, as well as mixtures thereof. Combinations of water and one or more of the mentioned organic solvents also can be employed.

The alkaline base can be chosen from among various hydroxides, carbonates, phosphates and amines. Illustrative bases include the inorganic bases such as sodium hydroxide, potassium hydroxide, sodium carbonate, potassium carbonate, sodium bicarbonate, potassium bicarbonate, and the like, and organic bases such as alkyl amines, and include, but are not limited to, diethylamine, triethylamine, and the like.

In aqueous media the pH is typically between approximately 10 and 14 with a pH of about 12 to about 13.5 being the preferred range. The hydrolysis reaction is typically carried out at a temperature of from about 20°C to about 80°C, preferably about 50°C to 60°C for a period of about 10 to about 30 minutes. For example, the hydrolysis can be conducted in 3% triethylamine in water at room temperature (22°-25°C) for a period of 48 hours. The only requirement in the choice of temperature and time of hydrolysis is that deacylation occurs to remove only the β-hydoxymyristic at position 3.

In practice it has been found that a particularly desirable hydrolysis method involves dissolving lipid A or monophosphoryl lipid A in chloroform:methanol 2:1 (v/v), saturating this solution with an aqueous buffer consisting of 0.5 M $Na_2CO_3$ at pH 10.5, and then flash evaporating the solvent at 45°-50°C under a vacuum or an aspirator (approximately 100 mm Hg). The resulting material is selectively deacylated at position 3. This process can also be carried out with any of the inorganic bases listed above. The addition of a phase transfer catalyst, such as tetrabutyl ammonium bromide, to the organic solution prior to saturation with the aqueous buffer may be desirable in some cases.

In preparing the composition of the subject invention, generally, the attenuated lipid A derivative (ALD) is combined with the surfactant each being dissolved in a solvent. The solvent is evaporated leaving a film. Water is added to the film and the resulting suspension is sonicated while heated until clear. The final suspension has a particle size of approximately 40-150 nm and preferably from about 50 to about 70 nm.

The ALD and surfactant are combined at a molar ratio of about 10 parts ALD to from about 1 part to about 5 parts surfactant. Preferably, the components are combined in a molar ratio of about 4 parts ALD to 1 part surfactant. Surfactants useful according to the subject invention include but are not limited to bile salts, natural phospholipids and sphingolipids. Bile salts such as glycodeoxycholate and deoxycholate are useful as surfactants in the claimed compositions. Other suitable surfactants include sphingolipids such as sphingomyelin and sphingosine and phospholipids such as egg phosphatidylcholine, 1,2-Dimyristoyl-sn-glycero-3-phosphoethanolamine, L-α-Phosphatidylethanolamine, and 1,2-Dipalmitoyl-sn-glycero-3-phosphocholine or mixtures thereof. In a preferred embodiment, the phospholipid 1,2-Dipalmitoyl-sn-glycero-3-phosphocholine (DPPC) is the surfactant. DPPC is accepted for use in humans and is especially effective when the formulation is administered intranasally.

The ALD and surfactant are dissolved and thoroughly admixed in a solvent. Aqueous or organic solvents useful according to the subject invention include chloroform, alcohols (eg. ethanol), dimethyl sulfoxide (DMSO), dimethylformamide (DMF), and the like, as well as mixtures thereof.

The solvent is evaporated from the mixture of ALD and surfactant leaving a film. Water is added to the film and the resulting suspension is sonicated while heated until clear. It is preferred that the suspension be sonicated in a water bath sonicator. The water bath temperature can be from 40°C to 80°C and preferably about 60°C. The suspension can be sonicated for periods of 5 minutes to approximately one hour until clear. Periods of sonication will vary depending upon the volume and concentration of the suspension but can be readily determined by one skilled in the art. The final suspension has a particle size of approximately 40-150 nm and preferably from about 50 to about 70 nm.

An effective amount of the composition of the subject invention is administered to a warm-blooded animal with an antigen to enhance the immune response of the animal to that antigen. The composition of the subject invention enhances both the humoral immune response of an animal to an antigen as well as the cellular immune response. The amount of antigen administered to elicit the desired response can be readily determined by one skilled in the art and will vary with the type of antigen administered, route of administration and immunization schedules. For example, 0.1 μg of tetanus toxoid administered with the claimed composition subcutaneously to a mouse in two immunizations 21 days apart elicits a humoral immune response to that antigen. Administered intranasally, the composition of the subject invention and an antigen stimulate the production of cytotoxic T-lymphocytes. Hepatitis B surface antigen (2.5 μg) administered intranasally at days 0 and 21 in the claimed composition stimulated the production of cytotoxic T-lymphocytes in immunized animals. Further, the composition of the subject invention is particularly effective in eliciting an IgA response in immunized animals when administered intranasally. Mice administered 0.5-12.5 μg of tetanus toxoid in an aqueous formulation of 3-O-deacylated monophosphoryl lipid A (3D-MLA/AF) displayed increased IgA titers to that antigen. An effective amount of the composition of the subject invention is that amount which stimulates or enhances an immune response. For example, an effective amount of the claimed composition can contain from 1 to about 250 micrograms of attenuated lipid A derivative and preferably from about 25 to about 50 micrograms based upon administration to a typical 70 kg adult patient.

The following examples are offered to further illustrate but not limit both the compositions and the method of the present invention. It is to be understood that the mouse models presented herein are representative of warm blooded animals and correlate reasonably with events for other warm blooded animals, including humans. All percentages are by weight and all solvent mixture proportions are by volume unless otherwise noted.

Example 1-Preparation of an Aqueous Formulation of an Attenuated Lipid A Derivative.

An aqueous preparation of 3-O-deacylated monophosphoryl lipid A (3D-MLA/AF) according to the subject invention comprising 1000 µg/ml 3D-MLA (Ribi ImmunoChem Research, Inc., Hamilton, Montana 59840) , an attenuated form of lipid A from *Salmonella minnesota* R 595 and 118 µg/ml 1,2 Dipalmitoyl-sn-glycero-3-phosphocholine (DPPC) in Water for Injection was prepared as follows:

A solution of DPPC was prepared at a concentration of 4 mg/ml in ethanol and vortexed until clear. A 2.7 ml aliquot of the DPPC solution was added to a vial containing 100 mg lyophilized 3D-MLA and swirled gently to wet the 3D-MLA. The ethanol was removed by blowing a stream of filtered nitrogen gently into the vial. Water for Injection (91.7 ml) was added to the vial which was then stoppered, sealed and suspended in a Labline 9303 water bath sonicator. The suspension was sonicated for 10 minutes at 60°C until clear. The resulting aqueous formulation contained particles of 70 nm measured by a PSC100 Spectrometer from Malvem Instruments and was filter sterilized through a 0.2 µm filter.

Example 2-Stimulation of an Antibody Response.

Mice immunized with tetanus toxoid (TT) in the aqueous formulation of the subject invention generated tetanus toxoid specific antibody. The TT-specific total IgG titer and IgG isotypes (2a, 2b, 1) titers were measured by enzyme-linked immunosorbent assay (ELISA) in the sera of mice following immunization.

Female ICR mice were immunized with a dose of vaccine containing 0.1 µg of tetanus toxoid (TT) + 50 µg 3D-MLA/AF or 0.1 µg TT in saline. 3D-MLA/AF was prepared as in Example 1. The vaccines were administered by subcutaneous injection on days 0 and 21. Serum was collected 14 days post secondary immunization and assayed by standard ELISA techniques to report the relative amounts of tetanus-toxoid specific antibody of $IgG_1$, $IgG_{2a}$ and $IgG_{2b}$ isotypes as well as total IgG.

Figure 1 shows the tetanus toxoid specific antibody titer generated by 3D-MLA/AF. 3D-MLA/AF when administered with the tetanus toxoid antigen stimulates the production of IgG antibody in immunized animals and in particular actively stimulates $IgG_{2a}$ production.

Example 3-Stimulation of Cellular Proliferation.

Mice primed by immunization with the adjuvant composition of the subject invention and a purified protein derivative (PPD) (tuberculin) exhibited a proliferative response *in vitro* when spleen cells were treated with that antigen.

Female BALB/c mice were immunized by subcutaneous injection with a dose of vaccines containing 50 µg PPD + 50 µg 3D-MLA/AF. 3D-MLA/AF was prepared as in Example 1. Spleen cells were harvested 14 days after immunization and used as a source of lymphocytes in a proliferation assay. The spleen cells were cultured for 96 hr in microtiter wells at a concentration of $10^6$ cells/ml in media containing 0.1, 1 or 10 µg PPD/ml. Tritiated thymidine was added to the cultures during the final 24 hr of incubation. The cells were harvested on glass fiber filters and tritium incorporation was determined. Stimulation indices were determined by dividing counts per minute (CPM) of cells stimulated with PPD by the CPM of cells cultured in media alone. The resulting data are shown in Figure 2.

Example 4-Stimulation of a Cytotoxic T-lymphocyte Response.

The induction of a cytotoxic T-lymphocyte response after administration of the aqueous adjuvant composition of the subject invention and a protein antigen was detected by a cytotoxicity assay. Groups of C57/BL/6 mice were given a primary immunization subcutaneously (inguinal region) with 25 µg ovalbumin (OVA) formulated in 3D-MLA/AF. 3D-MLA/AF was prepared as in Example 1. The injected volume was 200 µl. Twenty-one days later three mice per experimental group were killed and spleens removed and pooled as single cell suspensions and counted.

Spleen cells (75 X $10^6$ cells in 3-4 ml media) from the experimental groups were placed in a 25 cm² T-flask.

Next, 1.0 ml of irradiated (20,000 rads) E.G7 (OVA) cells at 5 X $10^6$/ml were added to the flask. The volume was brought to 10 ml. The cultures were maintained by placing the T-flasks upright in a 37°C, 5% $CO_2$ incubator for four days. On day 4 the surviving cells were recovered from the flasks, washed 1X, resuspended in 5.0 ml, and counted.

Recovered effector cells were adjusted to 5 X $10^6$ viable cells/ml and 100 µl volumes were diluted serially in triplicate in wells of 96 well round-bottom plates (Corning 25850) using 100 µl/well of media as a diluent. Next, 100 µl volumes of $^{51}$Cr-labelled (see below) targets [E.G7 (OVA)-an ovalbumin gene transfected EL-4 cell line] at 1 X $10^5$ cells/ml were added to the wells. Spontaneous release (SR) wells contained 100 µl of targets and 100 µl of media. Maximal release (MR) wells contained 100 µl of targets and 100 µl detergent (2% Tween 20). Effector/target (E/T) ratios were 50:1, 25:1, 12.5:1, 6.25:1. The plates were centrifuged at 400 Xg and incubated at 37°C, 5% $CO_2$ for 4 hr. After the incubation the well supernatants were collected using a Skatron Supernatant Collection System.

$$\text{Percent specific lysis} = 100 \; X \left[ \frac{(Exp.Release \; - \; SR)}{(MR \; - \; SR)} \right]$$

Target cells, E.G7 (OVA), were labelled with $^{51}$Cr (sodium chromate) as follows. In a total volume of 1.0 ml were mixed 5 X $10^6$ target cells and 250 µCi $^{51}$Cr in 15 ml conical tube. The cell suspensions was incubated in a 37°C water bath for 90 min., with gentle mixing every 15 min. After incubation the labelled cells were washed 3X by centrifugation and decanting with 15 ml volumes of media. After the third centrifugation the cells were resuspended in 10 ml of fresh media and allowed to stand at room temperature for 30 min. and then centrifuged. The cells were finally resuspended in media to 1 X $10^5$ cells/ml. The results of the cytotoxicity assay are presented in Table 1.

Table 1

| Material | % Cytotoxicity ($^{51}$Cr-release) Effector: Target Ratio | | | |
|---|---|---|---|---|
| | 50:1 | 25:1 | 12.5:1 | 6.25:1 |
| PBS* | 13 | 10 | 7 | 2 |
| 3D-MLA/AF | 61 | 60 | 59 | 45 |
| Non-immune spleen cells | 8 | 4 | 2 | 2 |

*phosphate buffered saline

Example 5-Stimulation of an Antibody Response by Intranasal Administration of the Aqueous ALD formulation.

Mice administered tetanus-toxoid (TT) in 3D-MLA/AF intranasally produced IgA titers detectable in both serum and fecal extracts. Further, intranasal administration of the aqueous formulation of the subject invention and TT produced high titers of the IgG isotypes IgG$_{2a}$ and IgG$_{2b}$.

Groups of ICR mice were given intranasally, 0.5, 2.5, 10 or 12.5 µg tetanus toxoid in phosphate buffered saline (PBS) or admixed with 25 µg 3D-MLA/AF. 3D-MLA/AF was prepared as in Example 1. Mice were primed on day 0, bled on day 10 (d10P1°), boosted on day 14, bled on day 24 (d10P2°), boosted on day 28, bled on day 38 (d10P3°). ELISA for IgG- and IgA specific anti-tetanus toxoid antibody was done on pooled sera from each bleed. Fecal extracts were examined on day 22 (d7P2°). IgG and IgA titers of sera and fecal extracts of immunized mice are shown in Tables 2-5.

## Table 2

| | | Serum Anti-Tetanus Toxoid Titer[1] | | | | | |
|---|---|---|---|---|---|---|---|
| | | IgG-Specific | | | IgA-Specific | | |
| Vaccine* | Route | d10P1° | d10P2° | d10P3° | d10P1° | d10P2° | d10P3° |
| 0.5 µg TT + PBS | IN | 200 | 400 | 25,600 | <200 | <200 | <200 |
| 2.5 µg TT + PBS | IN | 400 | 51,200 | 25,600 | <200 | <200 | <200 |
| 12.5 µg TT + PBS | IN | 3,200 | 51,200 | 102,400 | <200 | 200 | 400 |
| 0.5 µg TT + 3D-MLA/AF | IN | 12,800 | >409,600 | >409,600 | <200 | 800 | 6,400 |
| 2.5 µg TT + 3D-MLA/AF | IN | 51,200 | >409,600 | >409,600 | <200 | 12,800 | 25,600 |
| 12.5µg TT + 3D-MLA/AF | IN | 102,400 | >409,600 | >409,600 | <200 | 25,600 | 102,400 |
| 0.5 µg TT + PBS | SQ+ | 800 | 204,800 | 409,600 | <200 | <200 | <200 |

*n=4

+ SQ=subcutaneous

EP 0 971 739 B1

Table 3

| IgG Isotype Analysis of Serum from d10P3° Bleeds in Table 2. | | | | |
|---|---|---|---|---|
| | | **Anti-Tetanus Toxoid Titer[1]** | | |
| **Vaccine** | **Route** | **IgG$_1$** | **IgG$_{2a}$** | **IgG$_{2b}$** |
| 0.5 µg TT + PBS | IN | 25,600 | 6,400 | 25,600 |
| 2.5 µg TT + PBS | IN | 51,200 | 3,200 | 25,600 |
| 12.5 µg TT + PBS | IN | 204,800 | 12,800 | 51,200 |
| 0.5 µg TT + 3D-MLA/AF | IN | 819,200 | 409,600 | 819,200 |
| 2.5 µg TT + 3D-MLA/AF | IN | >819,200 | 819,200 | >819,200 |
| 12.5µg TT + 3D-MLA/AF | IN | >819,200 | >819,200 | >819,200 |
| 0.5 µg TT + PBS | SQ | 819,200 | 6,400 | 25,600 |
| Normal Mouse Sera | --- | <400 | <400 | <400 |

EP 0 971 739 B1

## Table 4

### Serum Anti-Tetanus Toxoid Titer[1]

| Vaccine | Route | IgG-Specific | | IgA-Specific | | Fecal Extract d7P2° | |
| --- | --- | --- | --- | --- | --- | --- | --- |
| | | d10P2° | d10P3° | d10P2° | d10P3° | IgG | IgA |
| TT* + 3D-MLA/AF/PBS | IN | >102,400 | >>102,400 | 6,400 | 25,600 | <50 | 1,600 |
| TT + DPPC/PBS | IN | 6,400 | 6,400 | 100 | 200 | <50 | <50 |
| TT + 3D-MLA/AF/PBS | SQ | >102,400 | >102,400 | 100 | 100 | <50 | <50 |
| Normal Mouse Sera | --- | 50 | 50 | 100 | 100 | <50 | <50 |

* 10 µg of tetanus toxoid were administered

Table 5

| IgG Isotype Analysis of Serum from d10P3° Bleeds in Table 4. | | | | |
|---|---|---|---|---|
| | | **Anti-Tetanus Toxoid Titers[-1]** | | |
| **Vaccine** | **Route** | **IgG$_1$** | **IgG$_{2a}$** | **IgG$_{2b}$** |
| TT + 3D-MLA/AF/PBS | IN | >819,200 | 102,400 | 409,600 |
| TT + DPPC/PBS | IN | 25,600 | 1,600 | 3,200 |
| TT + 3D-MLA/AF/PBS | SQ | >819,200 | 51,200 | 102,400 |
| Normal Mouse Sera | --- | <400 | <400 | <400 |

Example 6-Stimulation of an Immune Response to Hepatitis B Surface Antigen by Intranasal Administration of the Aqueous ALD Formulation.

Mice administered hepatitis B surface antigen (HBSAG) in the composition of the subject invention intranasally produced serum IgG and IgA titers to that antigen. Secretory IgA was detected in vaginal washes and the induction of a cytotoxic T-lymphocyte response was detected by a cytotoxicity assay.

Groups of Balb/C mice were given a primary immunization (1 °) intranasally with 2.5 µg HBsAg + 10 µg 3D-MLA/AF in a volume of 20 µl. 3D-MLA/AF was prepared as in Example 1. Twenty-one days later mice were given a secondary immunization (2°) of 7.5 µg HBsAg + 10 µg 3D-MLA/AF intranasally in 20 µl. A tertiary immunization (3°) identical in composition to the secondary immunization was administered 28 days after the secondary immunization. Assays were conducted to detect cytotoxic T-lymphocyte activity at 16 days post secondary immunization (d16,post 2°) and 8 days post tertiary immunization (d8, post 3°). Serum and mucosal antibody titers were assessed at 22 days post secondary immunization (d22, post 2°) and 21 days post tertiary immunization (d21, post 3°). All assays were conducted by methods standard in the art and described in previous Examples 2 and 4. Results from this experiment are shown in Tables 6-8.

Table 6

| | | **% Cytotoxicity ($^{51}$Cr-release) Effector: Target Ratio** | | | |
|---|---|---|---|---|---|
| **Material** | **Day** | **50:1** | **25:1** | **12.5:1** | **6.25:1** |
| 3D-MLA/AF | d16, post 2° | 38 | 22 | 15 | 9 |
| Vehicle | | 3 | 2 | 0 | 0 |
| Non-immune spleen cells | | 3 | 3 | 0 | 0 |
| 3D-MLA/AF | d8, post 3° | 82 | 65 | 49 | 36 |
| Vehicle | | 5 | 2 | 1 | 1 |
| Non-immune spleen cells | | 7 | 5 | 3 | 3 |

Table 7

| | | **Anti HBsAg Titer[-1]** | | |
|---|---|---|---|---|
| **Material** | **Day** | **IgG$_1$** | **IgG$_{2a}$** | **IgA** |
| 3D-MLA/AF | d22, post 2° | 256,000 | 64,000 | 1,600 |
| Vehicle | | <2,000 | <2,000 | <200 |
| 3D-MLA/AF | d21, post 3° | 1,000,000 | 1,000,000 | 25,600 |
| Vehicle | | <2,000 | <2,000 | <200 |

Groups of Balb/C mice were immunized with 2.5 µg HBsAg + 10 µg 3D-MLA/AF intranasally and boosted intranasally with 7.5 µg HBsAg + 10 µg 3D-MLA/AF 21 days later. Vaginal samples were collected 10 days after the booster immunization.

Table 8

| Material | Vaginal Wash Anti-HBSAG Titer[1] | |
| | IgG | IgA |
| --- | --- | --- |
| 3D-MLA/AF | 100 | 6400 |
| Vehicle | <50 | <50 |

The intranasal administration ofHBsAg in the composition of the subject invention stimulated both a humoral and cellular immune response to that antigen. Intranasal immunization with the antigen formulated in 3D-MLA/AF induced a cytotoxic T-lymphocyte response and antigen specific humoral and mucosal immune responses.

Example 7-Generation of a Protective Immune Response to Influenza by Intranasal Administration of the Aqueous ALD Formulation.

Mice immunized intranasally with FLUSHIELD® influenza vaccine containing hemagglutinin antigen formulated in the composition of the subject invention produced both IgG and IgA which were recovered in vaginal washes. Immunized mice were also protected 100% from subsequent influenza challenge.

ICR mice were immunized three times at 21 day intervals intranasally with FLUSHIELD influenza vaccine (Wyeth-Lederle) containing 0.3 μg hemagglutinin antigen (HA) + 10 μg 3D-MLA/AF. 3D-MLA/AF was prepared as in Example 1. Vaginal washes were collected 14 days after the final immunization. Mice were challenged with 10 $LD_{50}$ (lethal dose 50) of infectious influenza A/HK/68 thirty-five days after the final immunization and monitored for mortality.

Table 9

| Group | IgA Vaginal Wash | IgG Vaginal Wash | % Protection |
| --- | --- | --- | --- |
| Nonimmune | <20 | <20 | 0 |
| Vehicle | 160 | 80 | 60 |
| 3D-MLA/AF | 2560 | 1280 | 100 |

Example 8-Compositions of Monophosphoryl Lipid A.

Monophosphoryl lipid A (MLA) can be formulated into the aqueous compositions of the subject invention and administered in the same quantities and amounts as in Examples 1-7 to produce similar results.

It is understood that the foregoing examples are merely illustrative of the present invention. Certain modifications of the compositions and/or methods employed may be made and still achieve the objectives of the inventions. Such modifications are contemplated as within the scope of the claimed invention.

**Claims**

1. An adjuvant composition comprising an adjuvant in water which further contains a surfactant, **characterised in that** the adjuvant consists of an attenuated lipid A derivative selected from monophosphoryl lipid A (MLA) or 3-O-deacylated monophosphoryl lipid A (3D-MLA), the water is free of co-solvent and **in that** the molar ratio of the MLA or 3D-MLA to the surfactant is from 10:1 1 to 2:1.

2. The composition of claim 1, wherein the attenuated lipid A derivative is MLA.

3. The composition of claim 1, wherein the attenuated lipid A derivative is 3D-MLA.

4. The composition of any of claims 1 to 3, wherein the surfactant is selected from glycodeoxycholate, deoxycholate, sphingomyelin, sphingosine, phosphatidylcholine, 1,2-dimyristoyl-sn-glycero-3-phosphoethanolamine, L-α-phosphatidylethanolamine and 1,2-dipalmitoyl-sn-glycero-3-phosphocholine, or a mixture thereof.

5. The composition of claim 4, wherein the surfactant is 1,2-dipalmitoyl-sn-glycero-3-phosphocholine.

6. The composition of any of claims 1 to 5, wherein the molar ratio of the attenuated lipid A derivative to surfactant is 4:1.

7. The composition of any of claims 1 to 6 which is a clear aqueous suspension of the MLA or 3D-MLA and the surfactant.

8. The composition of claim 7, wherein the suspension has particles of a size of between 50 nm and 70 nm.

9. The composition of any of claims 1 to 8 which consists of the MLA or 3D-MLA, the surfactant and water.

10. The composition of any of claims 1 to 9 which is obtainable by:

   combining the MLA or 3D-MLA and the surfactant, the MLA or 3D-MLA and the surfactant being dissolved and thoroughly mixed in a solvent;
   evaporating the solvent;
   adding water and resuspending the MLA or 3D-MLA and surfactant;
   sonicating the suspension while heating until clear.

11. A vaccine comprising an adjuvant in a solvent, wherein the vaccine comprises the composition of any of claims 1 to 10, the adjuvant consists of the MLA or 3D-MLA and the solvent is water free of a co-solvent.

12. The vaccine of claim 11 which consists of the composition of any of claims 1 to 11 and an antigen.

13. A method of making an aqueous adjuvant composition, comprising:

   a) dissolving one or more surfactants in a solvent;
   b) mixing the dissolved surfactants with an attenuated lipid A derivative (ALD) selected from monophosphoryl lipid A (MLA) or 3-O-deacylated monophosphoryl lipid A (3D-MLA);
   c) evaporating the solvent from the resulting mixture;
   d) adding water to the evaporated mixture, to obtain a suspension of the ALD in water in a molar ratio (ALD: surfactant) of from 10:1 1 to 2:1; and
   e) heating and sonicating the suspension until clear.

14. The method of claim 13, wherein the surfactant is selected from glycodeoxycholate, deoxycholate, sphingomyelin, sphingosine, phosphatidylcholine, 1,2-dimyristoyl-sn-glycero-3-phosphoethanolamine, L-$\alpha$-phosphatidylethanolamine and 1,2-dipalmitoyl-sn-glycero-3-phosphocholine, or a mixture thereof.

15. The method of claim 13 or claim 14, wherein the molar ratio of the attenuated lipid A derivative to surfactant is 4:1.

16. The method of any of claims 13 to 15, wherein the end product is a clear aqueous suspension of the MLA or 3D-MLA and the surfacant.

17. The method of any of claims 13 to 16, wherein the solvent is selected from chloroform, alcohols, dimethyl sulfoxide, dimethyl formamide and mixtures thereof, e.g. ethanol.

18. The method of any of claims 13 to 17, wherein the suspension is heated to from 60°C to 80°C, e.g. 60°C.

19. The method of any of claims 13 to 18, wherein the suspension is sonicated for 5 to 60 minutes, e.g. 10 minutes.

20. The use of the method of any of claims 13 to 19 in the preparation of a vaccine.

21. The use of the composition of any of claims 1 to 10 for the manufacture of a medicament for enhancing the immune response, or the serum and mucosal secretory IgA response, of a warm-blooded animal to a protein antigen which is capable of eliciting an immune response in the animal.

22. The use, for the manufacture of a medicament which is a clear, co-solvent free, aqueous liquid, of MLA or 3D-MLA, water and a surfactant, the MLA or 3D-MLA being in a molar ratio to the surfactant of from 10:1 1 to 2:1.

**23.** The use of claim 22, wherein the medicament is a vaccine.

**Patentansprüche**

**1.** Zusatzstoff-Zusammensetzung, umfassend einen Zusatzstoff in Wasser, das weiter ein oberflächenaktives Mittel enthält, **dadurch gekennzeichnet, dass** der Zusatzstoff aus einem attenuierten Lipid A-Derivat besteht, das gewählt ist aus Monophosphoryl-Lipid A (MLA) oder 3-O-deacyliertem Monophosphoryl-Lipid A (3D-MLA), dass das Wasser frei von Co-Lösungsmitteln ist und dass das Molverhältnis des MLA oder 3D-MLA zu dem oberflächenaktiven Mittel im Bereich von 10 : 1 bis 2 : 1 liegt.

**2.** Zusammensetzung nach Anspruch 1, worin das attenuierte Lipid A-Derivat MLA ist.

**3.** Zusammensetzung nach Anspruch 1, worin das attenuierte Lipid A-Derivat 3D-MLA ist.

**4.** Zusammensetzung nach einem der Ansprüche 1 bis 3, worin das oberflächenaktive Mittel gewält ist aus Glycodeoxycholat, Deoxycholat, Sphingomyelin, Sphingosin, Phosphatidylcholin, 1,2-Dimyristoyl-sn-glycero-3-phosphoethanolamin, L-$\alpha$-Phosphatidylethanolamin und 1,2-Dipalmitoyl-snglycero-3-phosphocholin oder einer Mischung daraus.

**5.** Zusammensetzung nach Anspruch 4, worin das oberflächenaktive Mittel 1,2-Dipalmitoyl-sn-glycero-3-phosphocholin ist.

**6.** Zusammensetzung nach einem der Ansprüche 1 bis 5, worin das Molverhältnis des attenuierten Lipid A-Derivats zu dem oberflächenaktiven Mittel 4 : 1 ist.

**7.** Zusammensetzung nach einem der Ansprüche 1 bis 6 welche eine klare wässrige Suspension des MLA oder 3D-MLA und des oberflächenaktiven Mittels ist.

**8.** Zusammensetzung nach Anspruch 7, worin die Suspension Teilchen einer Größe von zwischen 50 nm und 70 nm aufweist.

**9.** Zusammensetzung nach einem der Ansprüche 1 bis 8, welche aus dem MLA oder 3D-MLA, dem oberflächenaktiven Mittel und Wasser besteht.

**10.** Zusammensetzung nach einem der Ansprüche 1 bis 9, welche erhältlich ist durch:

- Zusammengeben des MLA oder 3D-MLA und des oberflächenaktiven Mittels, wobei das MLA oder 3D-MLA und das oberflächenaktive Mittel in einem Lösungsmittel gelöst und sorgfältig darin gemischt werden;
- Verdampfen des Lösungsmittels;
- Zusetzen von Wasser und Resuspendieren des MLA oder 3D-MLA und des oberflächenaktiven Mittels;
- Behandeln der Suspension im Ultraschall unter Erhitzen, bis sie klar ist.

**11.** Impfstoff, umfassend einen Zusatzstoff in einem Lösungsmittel, worin der Impfstoff die Zusammensetzung nach einem der Ansprüche 1 bis 10 umfaßt, der Zusstzstoff aus dem MLA oder 3D-MLA besteht und das Lösungsmittel Wasser ist, das frei von einem Co-Lösungsmittel ist.

**12.** Impfstoff nach Anspruch 11, welcher aus der Zusammensetzung nach einem der Ansprüche 1 bis 11 und einem Antigen besteht.

**13.** Verfahren zur Herstellung einer wässrigen Zusatzstoff-Zusammensetzung, umfassend:

(a) das Lösen von einem oder mehreren oberflächenaktiven Mittel(n) in einem Lösungsmittel;
(b) das Mischen der gelösten oberflächenaktiven Mittel mit einem attenuierten Lipid A-Derivat (ALD), das gewählt ist aus Monophosphoryl-Lipid A (MLA) oder 3-O-deacyliertem Monophosphoryl-Lipid A (3D-MLA);
(c) das Verdampfen des Lösungsmittels von der resultierenden Mischung;
(d) das Zusetzen von Wasser zu der dem Verdampfungsvorgang unterworfenen Mischung unter Erhalt einer Suspension des ALD in Wasser in einem Molverhältnis (ALD: oberflächenaktives Mittel) von 10 : 1 bis 2 : 1; und

(e) das Erhitzen und Ultraschall-Behandeln der Suspension, bis sie klar ist.

**14.** Verfahren nach Anspruch 13, worin das oberflächenaktive Mittel gewählt ist aus: Glycodeoxycholat, Deoxycholat, Sphingomyelin, Sphingosin, Phosphatidylcholin, 1,2-Dimyristoyl-sn-glycero-3-phosphoethanolarnin, L-α-Phosphatidylethanolamin und 1,2-Dipalmitoyl-sn-glycero-3-phosphocholin oder einer Mischung daraus.

**15.** Verfahren nach Anspruch 13 oder Anspruch 14, worin das Molverhältnis des attenuierten Lipid A-Derivats zum oberflächenaktiven Mittel 4 : 1 ist.

**16.** Verfahren nach einem der Ansprüche 13 bis 15, worin das Endprodukt eine klare wässrige Suspension des MLA oder 3D-MLA und des oberflächenaktiven Mittels ist.

**17.** Verfahren nach einem der Ansprüche 13 bis 16, worin das Lösungsmittel gewählt ist aus Chloroform, Alkoholen, Dimethylsulfoxid, Dimethylformamid und Mischungen daraus, z.B. Ethanol.

**18.** Verfahren nach einem der Ansprüche 13 bis 17, worin die Suspension erhitzt wird auf 60 °C bis 80 °C, z.B. 60 °C.

**19.** Verfahren nach einem der Ansprüche 13 bis 18, worin die Suspension die Zeit von 5 bis 60 Minuten Ultraschall-behandelt wird, z.B. 10 Minuten.

**20.** Verwendung des Verfahrens nach einem der Ansprüche 13 bis 19 bei der Herstellung eines Impfstoffs.

**21.** Verwendung der Zusammensetzung nach einem der Ansprüche 1 bis 10 zur Herstellung eines Arzneimittels zur Verstärkung der Immunantwort oder der sekretorischen IgA-Antwort im Serum und den Schleimhäuten bei einem warmblütigen Tier gegenüber einem Protein-Antigen, das in der Lage ist, eine Immun-Antwort in dem Tier hervorzurufen.

**22.** Verwendung von MLA oder 3D-MLA, Wasser und einem oberflächenaktivem Mittel zur Herstellung eines Medikaments das eine klare, von Co-Lösungsmittel freie, wässrige Flüssigkeit ist, wobei das MLA oder 3D-MLA in einem Molverhältnis zu dem oberflächenaktiven Mittel von 10 : 1 bis 2 : 1 vorliegt.

**23.** Verwendung nach Anspruch 22, worin das Arzneimittel ein Impfstoff ist.

**Revendications**

**1.** Composition d'adjuvant comprenant un adjuvant dans l'eau qui contient de plus un surfactant, **caractérisée en ce que** l'adjuvant consiste en un dérivé de lipide A atténué choisi parmi le lipide A monosphoryle (MLA) ou lipide A monophosphoryle 3-O déacylaté(3D-MLA), **en ce que** l'eau ne contient pas de cosolvant et **en ce que** le rapport molaire du MLA ou 3D-MLA sur le surfactant est de l'ordre de 10:1 à 2:1.

**2.** Composition selon la revendication 1, **caractérisée en ce que** le dérivé de lipide A atténué est MLA.

**3.** Composition selon la revendication 1, **caractérisée en ce que** le dérivé de lipide A atténué est 3D-MLA.

**4.** Composition selon l'une des revendications 1 à 3, **caractérisée en ce que** le surfactant est choisi parmi le glycodéoxycholate, le déoxycholate, le sphingomyéline, le sphingosine, le phosphatidylcholine, 1,2-dimyristoyl-sn-glycéro-3-phosphoéthanolamine, L-α-phosphatidyléthanolamine et 1,2-dipalmitoyl-sn-glycéro-3-phosphocoline, ou un mélange de ceux-ci.

**5.** Composition selon la revendication 4, **caractérisée en ce que** le surfactant est 1,2-dipalmitoyl-sn-glycéro-3-phosphocoline.

**6.** Composition selon l'une des revendications 1 à 5, **caractérisée en ce que** le rapport molaire du dérivé de lipide A atténué sur le surfactant est de 4 : 1.

**7.** Composition selon l'une des revendications 1 à 6 qui est une suspension aqueuse claire de MLA ou 3D-MLA et du surfactant.

**8.** Composition selon la revendication 7, **caractérisée en ce que** la suspension a des particules d'une taille comprise entre 50nm et 70 nm.

**9.** Composition selon l'une des revendications 1 à 8, qui consiste en du MLA ou 3D-MLA, surfactant et eau.

**10.** Composition selon l'une des revendications 1 à 9 qui est obtenable en :

combinant le MLA et 3D-MLA et le surfactant, le MLA ou 3D-MLA et le surfactant étant dissous et mélangés totalement dans un solvant ;
évaporant le solvant ;
ajoutant l'eau et resuspendant le MLA ou 3D-MLA et le surfactant ;
sonifiant la molécule jusqu'à ce qu'elle devienne claire.

**11.** Vaccin comprenant un adjuvant dans un solvant, **caractérisé en ce que** le vaccin comprend la composition selon l'une des revendications 1 à 10, l'adjuvant consiste en du MLA ou 3D-MLA et le solvant est une eau ne contenant pas un cosolvant.

**12.** Vaccin selon la revendication 11 qui consiste en la composition selon l'une des revendications 1 à 11 et d'un antigène.

**13.** Méthode de fabrication d'une composition d'adjuvants aqueuse, comprenant :

a) la dissolution d'un ou plusieurs surfactants dans un solvant ;
b) mélange des surfactants dissous avec un dérivé lipide A atténué (ALD) choisi parmi le lipide A monophosphoryle (MLA) ou lipide A (3D-MLA) lipide 3-O-déacylaté ;
c) évaporation du solvant issu du mélange résultant ;
d) ajout d'eau au mélange évaporé, pour obtenir une suspension d'ALD dans l'eau dans un rapport molaire (ALD : surfactant) d'environ 10 :1 à 2 :1 ; et
e) le chauffage et la sonification de la suspension jusqu'à ce qu'elle soit claire.

**14.** Méthode selon la revendication 13, **caractérisée en ce que** le surfactant est choisi parmi le glycodéoxycholate, le déoxycholate, le sphingomyéline, le sphingosine, le phosphatidylcholine, 1,2-dimyristoyl-sn-glycéro-3-phosphoéthanolamine, L-$\alpha$-phosphatidyléthanolamine et 1,2-dipalmitoyl-sn-glycéro-3-phosphocoline, ou un mélange de ceux-ci.

**15.** Méthode selon la revendication 13 ou 14, **caractérisée en ce que** le rapport molaire du dérivé lipide A atténué sur le surfactant est 4 : 1.

**16.** Méthode selon l'une des revendications 13 à 15, **caractérisée en ce que** le produit final est une suspension aqueuse claire de MLA ou 3D-MLA et de surfactant.

**17.** Méthode selon l'une des revendications 13 à 16, **caractérisée en ce que** le solvant est choisi parmi le chloroforme, les alcools, sulfoxyde de diméthyle, formamide de diméthyle et des mélanges de ceux-ci, par exemple l'éthanol.

**18.** Méthode selon l'une des revendications 13 à 17, **caractérisée en ce que** la suspension est chauffée jusqu'à environ 60°C à 80°C, par exemple 60°C.

**19.** Méthode selon l'une des revendications 13 à 18, **caractérisée en ce que** la suspension est sonifiée pendant 5 à 60 minutes, par exemple 10 minutes.

**20.** Utilisation de la méthode selon l'une des revendications 13 à 19 dans la préparation d'un vaccin.

**21.** Utilisation de la composition selon l'une des revendications 1 à 10 pour la fabrication d'un médicament pour l'amélioration de la réponse immunitaire, ou la réponse IgA de sécrétion mucosale ou de sérum d'un animal à sang chaud pour un antigène de protéines qui est apte à provoquer une réponse immédiate dans l'animal.

**22.** Utilisation pour la fabrication d'un médicament qui est un liquide aqueux, sans cosolvant, clair, de MLA ou 3D-MLA, d'eau et d'un surfactant, le MLA ou 3D-MLA étant dans le rapport molaire sur le surfactant d'environ 10 :1 à 2 :1.

**23.** Utilisation selon la revendication 2, **caractérisée en ce que** le médicament est un vaccin.

Fig. 1(A)

Fig. 1(B)

Fig. 1(C)

Fig. 1(D)

Fig. 2